Europäisches Patentamt

European Patent Office

Office européen des brevets

(11) Veröffentlichungsnummer: **0 253 212 B1**

(12) **EUROPÄISCHE PATENTSCHRIFT**

(45) Veröffentlichungstag der Patentschrift: **22.01.92**

(21) Anmeldenummer: **87109499.1**

(22) Anmeldetag: **02.07.87**

(51) Int. Cl.⁵: **A23K 1/16, A61K 9/14**

(54) Verfahren zur Herstellung eines staubarmen freifliessenden Cholinchloridpulvers.

(30) Priorität: **16.07.86 DE 3623922**

(43) Veröffentlichungstag der Anmeldung:
**20.01.88 Patentblatt 88/03**

(45) Bekanntmachung des Hinweises auf die
Patenterteilung:
**22.01.92 Patentblatt 92/04**

(84) Benannte Vertragsstaaten:
**BE DE FR GB NL**

(56) Entgegenhaltungen:
**EP-A- 0 158 120**
**DD-A- 84 552**
**DE-A- 2 209 477**
**GB-A- 1 008 562**
**GB-A- 1 095 985**

**CHEMICAL ABSTRACTS, Band 99, Nr. 3, 18.**
**Juli 1983, Seiten 510,511, Zusammenfassung**
**Nr. 21183w, Columbus, Ohio, US; & HU-A-2**
**4266 (PHYLAXIA OLTOANYAG- ES TAPSZER-**
**TERMELO VALLALAT) 28-01-1983**

(73) Patentinhaber: **BASF Aktiengesellschaft**
**Carl-Bosch-Strasse 38**
**W-6700 Ludwigshafen(DE)**

(72) Erfinder: **Bayer, Robert**
**Steinsfurter Strasse 2 a**
**W-6921 Steinsfurt(DE)**
Erfinder: **Boettger, Guenter, Dr.**
**Langen Wingert 16 b**
**W-6702 Bad Duerkheim(DE)**
Erfinder: **Hiller, Rainer, Dr.**
**Linckensstrasse 85**
**W-4400 Muenster(DE)**
Erfinder: **Huber, Michael**
**Speyerer Strasse 9**
**W-6707 Schifferstadt(DE)**
Erfinder: **Koernig, Wolfgang, Dr.**
**Steige 7**
**W-6906 Leimen(DE)**
Erfinder: **Fritz, Wolfgang, Dr.**
**Wehlauerstrasse 129**
**W-7500 Karlsruhe(DE)**

## Beschreibung

Die Erfindung betrifft die Herstellung von staubarmen freifließendem Cholinchloridpulver auf Basis von Naturträgern oder Kieselsäure mit kontrollierter Teilchengrößenverteilung.

Cholin gehört zur Gruppe der Vitamine des B-Komplex und wird vielfach zur Anreicherung von Futtermittel verwendet. Da es sich in freier Form nicht handhaben und verwenden läßt, werden meist die Salze des Cholins, insbesondere Cholinchlorid, hergestellt. Cholin ist im Stoffwechsel der Fette unentbehrlich. Cholinmangel führt bei mehreren Tierarten anfangs zu Fettleber, Nierenschäden und später zu Leberzirrhose. Bei Geflügel z.B. kommt es zu Mißbildungen an Gelenken und Knochen, zu verzögertem Wachstum und erhöhter Sterblichkeit.

Cholinchlorid hat als reiner Feststoff eine extrem große Hygroskopizität. Das Produkt zieht bei Kontakt mit Umgebungsluft sofort Wasserdampf an und verflüssigt sich. Aus diesem Grunde kann es als Reinsubstanz nicht ver kauft werden. Als Futtermittelzusatz wird Cholinchlorid entweder in gelöster Form (75 bis 78 %ige wäßrige Lösung) oder als 50 %iges Trockenpulver mit einem anorganischen bzw. organischen Trägerstoff ("Adsorbat") verwendet. Als anorganischer Träger für Cholinchlorid ist hochporöse Kieselsäure bekannt, die mit Cholinchlorid vermischt nach dem Trocknen ein trockenes und rieselfähiges Pulver ergibt, das in dieser Form verkauft wird. Als organische Träger finden stark saugfähige, vermahlene Naturprodukte wie Maisspindelmehl, Reisschalen, Malzkeime, aufgepuffte Stärke aus Weizen, Mais usw. Anwendung.

Cholinchlorid auf Trägermaterialien wird in erster Linie beim Kleinverbraucher eingesetzt. Dieser mischt das Tierfutter vor Gebrauch entsprechend zusammen. Dabei wird in der Regel ohne Ausschluß der Luftfeuchtigkeit gearbeitet, d.h. Säcke, die nicht leer werden, bleiben bis zum nächsten Mal meist unverschlossen liegen.

Da das Cholinchlorid im Verhältnis von nur etwa 1:1000 dem Mischfutter beigefügt wird, bestehen für die Partikelgröße enge Grenzen: Bei zu groben Partikeln ist die Wahrscheinlichkeit, daß alle Tiere, insbesondere Küken, gleichviel Wirkstoff aufnehmen, nicht mehr gegeben, bei zu kleinen Partikeln ist die Lagerfähigkeit sowie die Handhabung erschwert. Schließlich besteht die Gefahr der Entmischung im Futter. Aus diesen Gründen muß die Partikelgröße in bestimmten Grenzen liegen.

Aus der US-PS 2 879 161 ist bekannt, daß sich Kieselsäure als Trägermaterial für Cholinchlorid sehr gut eignet, da sie infolge ihres außerordentlich hohen Adsorptionsvermögens zu zu 65 Gew.% Cholinchloridlösung beim Mischen aufnehmen kann. Nach dem Mischvorgang erhält man ein weißes, pulverförmiges und rieselfähiges Endprodukt mit etwa 50 Gew.% Cholinchlorid, 33 Gew.% Kieselsäure und 17 % Wasser.

Dieses Verfahren hat jedoch den Nachteil, daß die Konzentration von nur 50 Gew.% Cholinchlorid im Endprodukt relativ gering ist.

Es ist bereits auch ein Verfahren zur Herstellung pulverförmigen Cholinchlorids bekannt, bei dem als Träger für Cholinchlorid stärkehaltiger Mais verwendet wird. Der gemahlene Mais wird in einem Extruder unter Druck und Temperatur so vorbehandelt, daß er als aufgeblähter, poröser Strängling vorliegt. Das Extrudat wird mit 70 %iger wäßrige Cholinchloridlösung gemischt, auf eine Feuchte von 12 bis 14 % getrocknet und gemahlen. Das fertige Konzentrat enthält bis zu 50 Gew.% Cholinchlorid (UdSSR-Patent 698 606).

Dieses Verfahren jedoch hat folgende Nachteile:

a) Der Naturträger muß aufwendig und teuer vorbereitet werden.

b) Als Träger wird vorzugsweise nur Mais allein eingesetzt.

c) Die Restfeuchte von 12 bis 14 % Wasser nach der Trocknung ist hoch - dafür ist die Konzentration von nur 50 Gew.% Cholinchlorid gering.

d) Das Kornspektrum des Endproduktes ist sehr fein; dadurch besteht die Gefahr der Entmischung und durch zu rasche Feuchteaufnahme verschlechtert sich das Lager- und Fließverhalten.

Es bestand daher die Aufgabe, Cholinchloridpulver auf Kieselsäure oder Naturträger vorzuschlagen, die die genannten Nachteile nicht haben.

Diese Aufgabe zur Herstellung eines staubarmen, freifließenden Cholinchloridpulvers auf Basis von Naturträgern oder Kieselsäure, wobei der Cholinchloridgehalt auf Naturträgern 30 bis 60 Gew.% und auf Kieselsäure 30 bis 80 Gew.%, bezogen auf das Cholinchloridpulver beträgt, wird erfindungsgemäß dadurch gelöst, daß man

a) Cholinchloridlösung mit dem Träger im gewünschten Mengenverhältnis innerhalb des angegebenen Bereiches mischt,

b) die erhaltene Feuchtgutmischung kontinuierlich im Falle von Kieselsäure über eine Zweistoffdüse mit Druckgas und im Falle von Naturträgern über eine langsamdrehende Doppelpaddelschnecke in ein Wirbelbett, das bereits getrocknete Anteile an Cholinchloridpulver enthält, einführt und dort bei Temperaturen von 90 bis 180 °C trocknet,

c) das getrocknete Endprodukt aus dem Wirbelbett abzieht und auf Temperaturen unter 50 °C abkühlt, und

d) die Korngröße des Cholinchloridpulvers auf einen Bereich von 100 μm bis 1000 μm einstellt, indem man nach Siebung gröbere Anteile durch Mahlen zerkleinert und feinere Anteile in das Wirbelbett zurückführt.

Mit dem erfindungsgemäßen Verfahren ist es möglich, eine Kieselsäure mit einer mittleren Korngröße Von etwa 80 μm und einer spezifischen Oberfläche von 190 m²/g einzusetzen. Das nach dem Mischen, Trocknen und Kühlen erzielte Endprodukt ist staubarm, freifließend, nicht hygroskopisch und hat vorzugsweise den Korngrößenbereich von 100 bis 1000 μm.

Es ist dabei erwünscht, daß man bei der Vermischung eine homogene Mischung von Trägermaterialien und Cholinchloridlösung (78 % Cholinchlorid, 22 % Wasser) erzielt, damit sich Cholinchlorid im getrockneten Endprodukt größtenteils im Inneren des Trägers befindet und somit ein Verkleben des Endproduktes infolge der hohen Hygroskopizität von Cholinchlorid verhindert wird. Bei Verwendung des anorganischen Trägers Kieselsäure wird die Herstellung einer homogenen Mischung, z.B. dadurch erreicht, daß 78 %ige Cholinchloridlösung mit der Kieselsäure bei einer Mischtemperatur von 20 bis 100˚C in einem Mischer suspendiert wird. Die Suspension hat z.B. eine Dichte von 1200 kg/m³ und eine Feuchte von etwa 22 % Wasser (bezogen auf Trockensubstanz). Die reine Mischzeit wird durch die Temperung der Suspension kleiner als 20 Minuten. Aus verfahrenstechnischen Gründen wird die Cholinchlorid/Kieselsäure-Mischung hydrophobiert. Das hydrophobe Ca-Stearat wird gemeinsam mit der Kieselsäure zu 0,5 % (bezogen auf Trockenprodukt) der Suspension untergemischt.

Mit dem erfindungsgemäßen Verfahren gelingt es, die Feuchgutmischung (Suspension aus Cholinchlorid, Kieselsäure und Wasser) in ein Wirbelbett kontinuierlich über eine Zweistoffdüse durch Druckgas einzusprühen und dort zu trocknen.

Mit der Erfindung kann die Wachstumsgeschwindigkeit der Granulate im Wirbelbett durch Wasserzugabe gesteuert und die Endkorngröße (100 <d < 1000 μm) durch kontinuierliche Korngrößensteuerung (Siebung und Grobgutzerkleinerung: Feinanteil sowie zerkleinertes Grobgut werden zum Wirbelbett zurückgefördert) erzielt werden.

Als Kieselsäuren kommen Vor allem hydrophile Kieselsäuren mit einer Korngröße von 40 bis 300 μm und einer Oberfläche von 100 bis 300 m²/g in Betracht.

Mit dem erfindungsgemäßen Verfahren ist es gleichermaßen möglich, verfügbare billige organische Trägerstoffe mit Korngrößen im Bereich von 80 % zwischen 100 μm und 800 μm einzusetzen. Das nach dem Tränken, Trocknen und Kühlen erzielte Konzentrat ist staubarm, freifließend, lagerstabil und hat eine homogene Wirkstoffverteilung. Dies bedeutet, daß der Cholinchloridanteil an der Oberfläche geringer als bei Produkten des Standes der Technik ist und daß daher die Produkte deutlich geringer hygroskopisch sind.

Als Naturträger sind z.B. Weizengrießkleie, Maisspindelmehl, Corn Cobs, Rübenschnitzel oder Reisschalen zu nennen. Bei Einsatz von z.B. Weizengrießkleie bewährt sich ein Kornspektrum von 200 bis 1000 μm bei einem Wassergehalt unter 15 %.

Die Herstellung einer rieselfähigen, homogene Mischung mit optimaler Wirkstoffverteilung im Naturträger wird z.B. im einzelnen dadurch erreicht, daß der Naturträger mit der z.B. 78 %igen Cholinchloridlösung in einem Mischer bei einer Temperatur von 60 bis 100˚C und einer möglichst kurzen Mischzeit von z.B. 20 Min. getränkt wird. Die Mischzeit und die Cholinchloridaufnahme des Naturträgers wird durch die Mischtemperatur gesteuert. Die Mischung hat eine Feuchte von 15 bis 19 Gew.%, ist locker und rieselfähig und der Wirkstoff ist vollkommen und homogen von organischen Träger aufgenommen.

Zum kontinuierlichen Eintragen der Feuchtgutmischung in einem Wirbelschichttrocknungsapparat verwendet man zweckmäßig eine drehzahlregelbare, langsamdrehende Doppelpaddelschnecke mit progressiver Steigung.

Für schlechter fließfähige oder rieselfähige feuchte Naturträgermischungen sind pneumatische oder mechanische Dispergierungsaggregate gegebenenfalls vorzuziehen.

Beispiel 1

100 kg Cholinchloridlösung (78 Gew.% Cholinchlorid, 22 Gew.% Wasser) werden in einem Mischer vorgelegt und unter Rühren werden 0,5 kg Ca-Stearat und 19 kg feinverteilte Kieselsäure (Sipernat® 22 Degussa) zudosiert. Nach einer Mischzeit von weniger als 20 Minuten und einer Mischungstemperatur von 60 bis 100˚C wird die Suspension in ein Wirbelbett konitinuierlich über eine Zweistoffdüse durch Druckgas dispergiert und dort getrocknet. Das getrocknete Endprodukt wird anschließend in einem Produktkühler auf eine Temperatur kleiner 50˚C abgekühlt. Man erhält ein staubarmes, freifließendes und in Umgebungsluft nicht verklebendes Cholinchloridpulver mit einem auf die Trockenmasse bezogenen Gehalt von 80 Gew.% Cholinchlorid auf Kieselsäure.

Beispiel 2

100 kg Weizengrießkleie werden in einem langsamdrehenden Mischer vorgelegt und 169,5 kg Cholinchloridlösung (78 Gew.% Cholinchlorid, 22 Gew.% Wasser) zudosiert. Nach einer Tränkzeit von weniger als 20 Minuten bei einer Mischtemperatur von 60 bis 100˚C wird das lockere, rieselfähige organische Feuchtgut in einen Zwischenbunker abgelassen und von dort kontinuierlich mit einer Doppelpaddelschnecke in einem Wirbelschicht-trocknungsapparatdispergiert. Das in der Wirbel-schicht getrocknete Konzentrat wird in einem Produktkühler auf eine Temperatur unterhalb 50°C abgekühlt, anschließend gesiebt und das anfallende Grobgut staubarm und schonend in einer Mühle zerkleinert.

Man erhält aus den Verfahren ein staubarmes, freifließendes Produkt mit homogener Wirkstoffverteilung und einem Kornspektrum von 90 % im Bereich zwischen 200 μm und 1000 μm.

Der Wirkstoffgehalt beträgt 60 Gew.%, bezogen auf die Trockenmasse des organischen Trägers.

**Patentansprüche**

1. Verfahren zur Herstellung eines staubarmen, freifließenden Cholinchloridpulvers auf Basis von Naturträgern oder Kieselsäuren, wobei der Cholinchloridgehalt auf Naturträgern 30 bis 60 Gew.% und auf Kieselsäuren 30 bis 80 Gew.%, bezogen auf das Cholinchloridpulver beträgt. dadurch gekennzeichnet, daß man
   a) Cholinchloridlösung mit dem Träger im gewünschten Mengenverhältnis innerhalb des angegebenen Bereichs mischt,
   b) die erhaltene Feuchtgutmischung kontinuierlich im Falle von Kieselsäuren über eine Zweistoffdüse mit Druckgas und im Falle von Naturträgern über eine langsamdrehende Doppelpaddelschnecke in ein Wirbelbett, das bereits getrocknete Anteile an Cholinchloridpulver enthält, einführt und dort bei Temperaturen von 90 bis 180°C trocknet,
   c) das getrocknete Endprodukt aus dem Wirbelbett abzieht und auf Temperaturen unter 50°C abkühlt, und
   d) die Korngröße des Cholinchloridpulvers auf einen Bereich von 100 μm bis 1000 μm einstellt, indem man nach Siebung gröbere Anteile durch Mahlen zerkleinert und feinere Anteile in das Wirbelbett zurückführt.

2. Verfahren nach Anspruch 1, dadurch gekennzeichnet, daß man als Kieselsäure freindisperse Kieselsäure mit einer mittleren Teilchengröße von 40 bis 300 μm und einer spezifischen Oberfläche von 100 bis 300 m²/g verwendet.

3. Verfahren nach Anspruch 1, dadurch gekennzeichnet, daß man als Naturträger zerkleinerte Rübenschnitzel. Weizengrießkleie, Maisspindelmehl, Corn Cobs oder Reisschalen mit 80 Gew.% einer Korngröße von 150 μm bis 800

μm verwendet.

4. Verfahren gemäß Anspruch 1, dadurch gekennzeichnet, daß die Korngröße von 90 Gew.% des Cholinchloridpulvers auf Basis Naturträger auf einen Bereich von 200 μm bis 1 000 μm eingestellt wird, indem man nach Siehung gröbere Anteile durch Mahlung zerkleinert und feinere Anteile in das Wirbelbett zurückführt.

**Claims**

1. A process for the preparation of a low-dust free-flowing choline powder based on natural carriers of silicas, the choline chloride content on natural carriers being from 30 to 60% by weight and that on silicas being from 30 to 80% by weight, based on the choline chloride powder, wherein
   a) choline chloride solution is mixed with the carrier in the desired ratio within the stated range,
   b) the resulting moist mixture is introduced continuously, via a two-material nozzle with compressed gas in the case of silicas and via a slowly rotating twin-blade screw in the case of natural carriers, into a fluidized bed already containing dry choline chloride powder and is dried there at from 90 to 180°C,
   c) the dried end product is removed from the fluidized bed and cooled to below 50°C, and
   d) the particle size of the choline chloride powder is brought to 100-1,000 μm by a procedure in which, after screening, oversize is comminuted by milling and undersize is recycled to the fluidized bed.

2. A process as claimed in claim 1, wherein the silica used is finely divided silica having a mean particle size of from 40 to 300 μm and a specific surface area of from 100 to 300 m²/g.

3. A process as claimed in claim 1, wherein comminuted beet chips, wheat bran, corncob flour, corncobs or rice husks in which 80% by weight of the particles have a size of from 150 to 800 μm is or are used as natural carriers.

4. A process as claimed in claim 1, wherein the particle size of 90% by weight of the choline chloride powder based on a natural carrier is brought to 200-1,000 μm by a procedure in which, after screening, oversize is comminuted by milling and undersize is recycled to the fluidized bed.

## Revendications

1. Procédé de préparation d'une poudre contenant du chlorure de choline ne formant pas de poussière et s'écoulant librement, à base de supports naturels ou d'acides siliciques, la teneur en chlorure de choline sur les supports naturels étant de 30 à 60% en poids et sur les acides siliciques étant de 30 à 80% en poids, par rapport à la poudre contenant du chlorure de choline, caractérisé en ce que

a) on mélange une solution de chlorure de choline avec le support dans le rapport en poids désiré compris dans la gamme de pourcentage sus-indiquée,

b) on introduit le mélange humide obtenu, en continu, dans le cas des acides siliciques par l'intermédiaire d'une buse pour deux substances avec un gaz sous pression et, dans le cas des supports naturels par l'intermédiaire d'une vis sans fin à double hélice tournant lentement, dans un lit fluidisé, qui contient déjà des fractions séchées de la poudre contenant du chlorure de choline, et dans lequel on sèche ledit mélange à des températures de 90 à 18° C,

c) on extrait du lit fluidisé le produit final séché et on le refroidit à des températures inférieures à 50° C, et,

d) on règle la taille des grains de la poudre contenant du chlorure de choline dans une gamme de 100 $\mu$m à 1000 $\mu$m, en brisant par broyage les fractions plus grosses après tamisage et en recyclant dans le lit fluidisé les fractions plus fines également après tamisage.

2. Procédé suivant la revendication 1, caractérisé en ce que l'on utilise, comme acide silicique, de l'acide silicique finement dispersé ayant une taille moyenne des particules de 40 à 300 $\mu$m et une surface spécifique de 100 à 300 m$^2$/g.

3. Procédé suivant la revendication 1, caractérisé en ce que l'on utilise, comme supports naturels, des cossettes de betteraves broyées, du son de blé, de la farine de maïs, des rafles de maïs ou des enveloppes de grains de riz, 80% en poids de ces particules ayant une taille comprise entre 150 $\mu$m et 800 $\mu$m.

4. Procédé suivant la revendication 1, caractérisé en ce que la taille des grains de 90% en poids de la poudre contenant du chlorure de choline et à base de supports naturels est réglée dans une gamme de 200 $\mu$m à 1000 $\mu$m, par pulvérisation par broyage des fractions plus grosses après tamisage et par recyclage dans le lit fluidisé des fractions plus fines également après tamisage